(19) Europäisches Patentamt
European Patent Office
Office européen des brevets

(11) Publication number: 0 285 356
A2

(12) EUROPEAN PATENT APPLICATION

(21) Application number: 88302740.1

(22) Date of filing: 28.03.88

(51) Int. Cl.[4]: C07D 405/06 , C07D 307/52 , C07D 307/56 , A61K 31/415

(30) Priority: 30.03.87 US 31735

(43) Date of publication of application: 05.10.88 Bulletin 88/40

(84) Designated Contracting States: AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Kruse, Lawrence Ivan
33 Firs Walk
Tewin Hertfordshire, AL7 1EY(GB)

(74) Representative: Giddings, Peter John, Dr. et al
Smith Kline & French Laboratories Ltd.
Corporate Patents Mundells
Welwyn Garden City Hertfordshire AL7 1EY(GB)

# (54) Dopamine-beta-hydroxylase inhibitors.

(57) Compounds of formula (I)

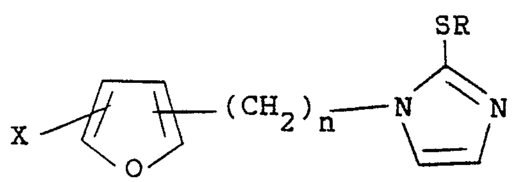

(I)

in which X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$ or $CO_2C_aH_{2a+1}$ wherein a is 1-5, or any accessible combination thereof of up to 3 substituents; R is H or $C_{1-4}$alkyl; and n is 1-5; or any pharmaceutically acceptable salt or hydrate thereof, processes for their preparation, intermediates useful in their preparation, pharmaceutical compositions containing them and their use in therapy as dopamine-$\beta$-hydroxylase inhibitors.

EP 0 285 356 A2

# DOPAMINE-$\beta$-HYDROXYLASE INHIBITORS

## FIELD OF THE INVENTION

This invention relates to novel compounds that inhibit dopamine-$\beta$-hydroxylase.

## BACKGROUND OF THE INVENTION

In the catecholamine biosynthetic pathway, tyrosine is converted in three steps to norepinephrine (NE). Intermediates are dihydroxyphenylalanine (DOPA) and dopamine (DA). Dopamine is hydroxylated to norepinephrine by dopamine-$\beta$-hydroxylase (DBH) in the presence of oxygen and ascorbic acid.

Inhibition of catecholamine activity decreases blood pressure. Weinshilboum, Mayo Clin. Proc. 55, 39 (1980), reviews compounds that inhibit catecholamine activity by acting upon adrenergic receptors. Alternatively, the catecholamine biosynthetic pathway can be suppressed at any of the three steps, resulting in reduced NE levels. In addition to producing an antihypertensive effect, inhibitors of NE synthesis are active as diuretics, natriuretics, cardiotonics, and vasodilators. Inhibition of DBH activity can have the added advantage of increasing DA levels, which as reported by Ehrreich et al., "New Antihypertensive Drugs," Spectrum Publishing, 1976, pp. 409-432, has selective vasodilator activity at certain concentrations.

DBH inhibitors also have been shown to reduce or prevent formation of gastric ulcers in rats by Hidaka et al., "Catecholamine and Stress," edit. by Usdin et al., Permagon Press, Oxford, 1976, pp. 159-165 and by Osumi et al., Japan J. Pharmacol. 23, 904 (1973).

A number of DBH inhibitors are known. These generally are divided into two classes, namely, metal chelating agents, which bind copper in the enzyme, and phenethylamine analogues. Rosenberg et al., "Essays in Neurochemistry and Neuropharmacology," Vol. 4, ed. by Youdim et al., John Wiley & Sons, 1980, pp. 179-192, and Goldstein, Pharmacol. Rev. 18(1), 77 (1966), review DBH inhibitors. The former report that many potent DBH inhibitors have a hydrophobic side chain of size comparable to the aromatic ring of DA, leading the authors to suggest that incorporation of a terminal hydroxyl group on a 4-to 6-carbon side chain on a phenethylalamine analogue may yield potent inhibitors.

Known DBH inhibitors include:

(a) 5-alkylpicolinic acids [See, Suda et al., Chem. Pharm. Bull. 17, 2377 (1969); Umezawa et al., Biochem. Pharmacol. 19, 35 (1969); Hidaka et al., Mol. Pharmacol. 9, 172 (1973); Miyano et al., Chem. Pharm. Bull. 26, 2328 (1978); Miyano et al., Heterocycles 14, 755 (1980); Claxton et al., Eur. J. Pharmacol. 37, 179 (1976)];

(b) BRL 8242 [See Claxton et al., Eur J. Pharmacol. 37, 179 (1976)];

(c) 1-alkylimidazole-2-thiols [See, Hanlon et al., Life Sci. 12, 417 (1973); Fuller et al., Adv. Enzyme Regul. 15, 267 (1976)];

(d) substituted thioureas [See, Johnson et al., J. Pharmacol. Exp. Ther. 168, 229 (1969)]; and

(e) benzyloxyamine and benzylhydrazine [See, Creveling et al., Biochim. Biophys. Acta 64, 125 (1962); Creveling et al., Biochim. Biophys. Acta 8, 215 (1962); Van Der Schoot et al., J. Pharmacol. Exp. Ther. 141, 74 (1963); Bloom, Ann. N.Y. Acad. Sci. 107, 878 (1963)].

All the above compounds except benzyloxyamine and benzylhydrazine apparently owe their inhibitory effect to metal chelating properties. Alkyl derivatives of imidazole-2-thiol are more potent, presumably due to non-specific interaction of the alkyl substituent with the enzyme. Benzyloxyamine and benzylhydrazine are phenethylalamine analogues which apparently act as competitive inhibitors.

In addition to the above compounds, Runti et al., Il Farmaco Ed. Sci. 36, 260 (1980), report that other fusaric acid derivatives and analogues inhibit DBH. These include phenylpicolinic acid, which has twice the inhibitory activity of fusaric acid, and 5-(4-chlorobutyl) picolinic acid, and others such as substituted amides of fusaric acid and acids and amides of 5-butyroylpicolinic acid, 5-aminopicolinic acid and 5-hydrazinopicolinic acid, and derivatives thereof.

Hidaka et al., Molecular Pharmacology, 9, 172-177 (1972) report that 5-(3,4-dibromobutyl)picolinic acid and 5-(dimethyldithiocarbamoylmethyl)picolinic acid are DBH inhibitors.

Bupicomide, 5-(n-butyl)picolinamine, is reported by Ehrreich et al., "New Antihypertensive Drugs", Spectrum Publications, 1976, pg. 409-432, to be a DBH inhibitor that has antihypertensive activity.

In European Patent Application No. 125,033 (published November 14, 1984) a series of 1-phenyl and 1-phenylalkylimidazole compounds having a mercapto or alkylthio group in the 2-position are disclosed.

These compounds are described as having DBH inhibiting activity.

United States Patent No. 4,487,761 describes several methylpyridine derivatives isolated from the fermentation broth of a strain of Streptoverticillium. These compounds inhibit DBH activity.

United States Patent No. 4,532,331 describes various 1-benzyl-2-aminomethylimidazole derivatives that inhibit DBH activity and includes pharmaceutical compositions containing these derivatives and methods of using these derivatives to inhibit DBH activity.

Non-specific, often toxic effects to known DBH inhibitors have obviated clinical use of these compounds. Fusaric acid, for example, is hepatotoxic. See, for example, Teresawa et al., Japan. Cir. J. 35, 339 (1971) and references cited therein. Presumably, the picolinic acid structure interacts with a number of metalloproteins and enzymes non-specifically to produce the observed side effects.

Recently, Skuballa, et al., discovered that certain heteroaromatic amines are DBH substrates and are potent, time-dependent DBH inhibitors. J. Med. Chem. 29:3, 315 (1986). Included among the heteroaromatic amines which inhibit DBH are 2-(2-furanyl)allylamine and 2-(3-furanyl)allylamine.

## SUMMARY OF THE INVENTION

The present invention resides in the discovery that DBH is inhibited by substituted 1-(2-or 3-furanylalkyl)imidazole-2-thiols and 1-(2-or 3-furanylalkyl)-2-imidazole-2-alkylthiols. These compounds are potent and produce prolonged DBH inhibition.

Presently preferred compounds of the invention and compounds included in the pharmaceutical compositions and used in the methods of the invention include:

1-(2-furanylmethyl)imidazole-2-thiol; and

1-(3-furanylmethyl)imidazole-2-thiol.

In a further aspect of the invention there are provided novel intermediates useful in preparing substituted 1-(2-or 3-furanylalkyl)imidazole-2-thiols and 1-(2-or 3-furanylalkyl)imidazole-2-alkylthiols.

The invention also is a method of inhibiting DBH activity in mammals, including humans, which comprises administering internally to a subject an effective amount of a substituted 1-(2-or 3-furanylalkyl)-imidazole-2-thiol of 1-(2-or 3-furanylalkyl)imidazole-2-alkylthiol.

Included in the present invention are pharmaceutical compositions comprising compounds useful in the method of the invention and a pharmaceutical carrier.

## DETAILED DESCRIPTION OF THE INVENTION

The presently invented compounds that inhibit DBH have the following formula:

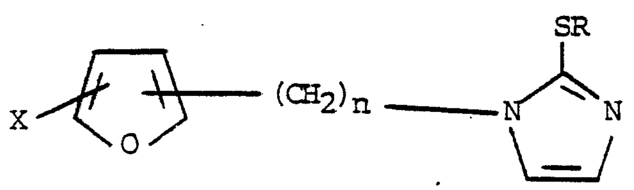

(I)

in which:

X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$, or $CO_2C_aH_{2a+1}$ wherein a is 1-5, or any accessible combination thereof of up to 3 substituents; or

n is 1-5;

R is hydrogen or $C_{1-4}$alkyl; or

any pharmaceutically acceptable salt or hydrate thereof.

As used herein, "accessible combination thereof" means any other combination of the substituents that is available by chemical synthesis and is stable, and "$C_{z-n}$alkyl" and "$C_{z-n}$alk" means a straight or branched hydrocarbon chain having from z to n carbon atoms.

It is intended that Formula I includes the tautomer of the compounds in which R is hydrogen, that is, compounds having the above formula wherein the imidazole moiety has either of the below formulae:

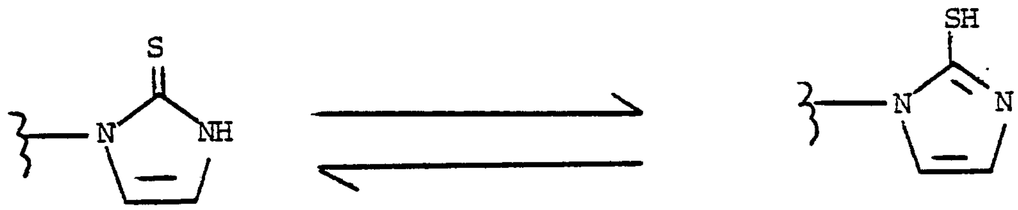

Formula (I) compounds are prepared from furanylaldehydes or furanylalkylaldehydes by processes exemplified in Scheme I, below. The starting furanylaldehyde and furanylalkylaldehydes are described in published references and can be purchased or readily prepared from available materials. In Scheme I, X and n are the same as in Formula (I). Y is $(OC_{1-4}alkyl)_2$ or $OC_{2-5}alkylO$.

Scheme I

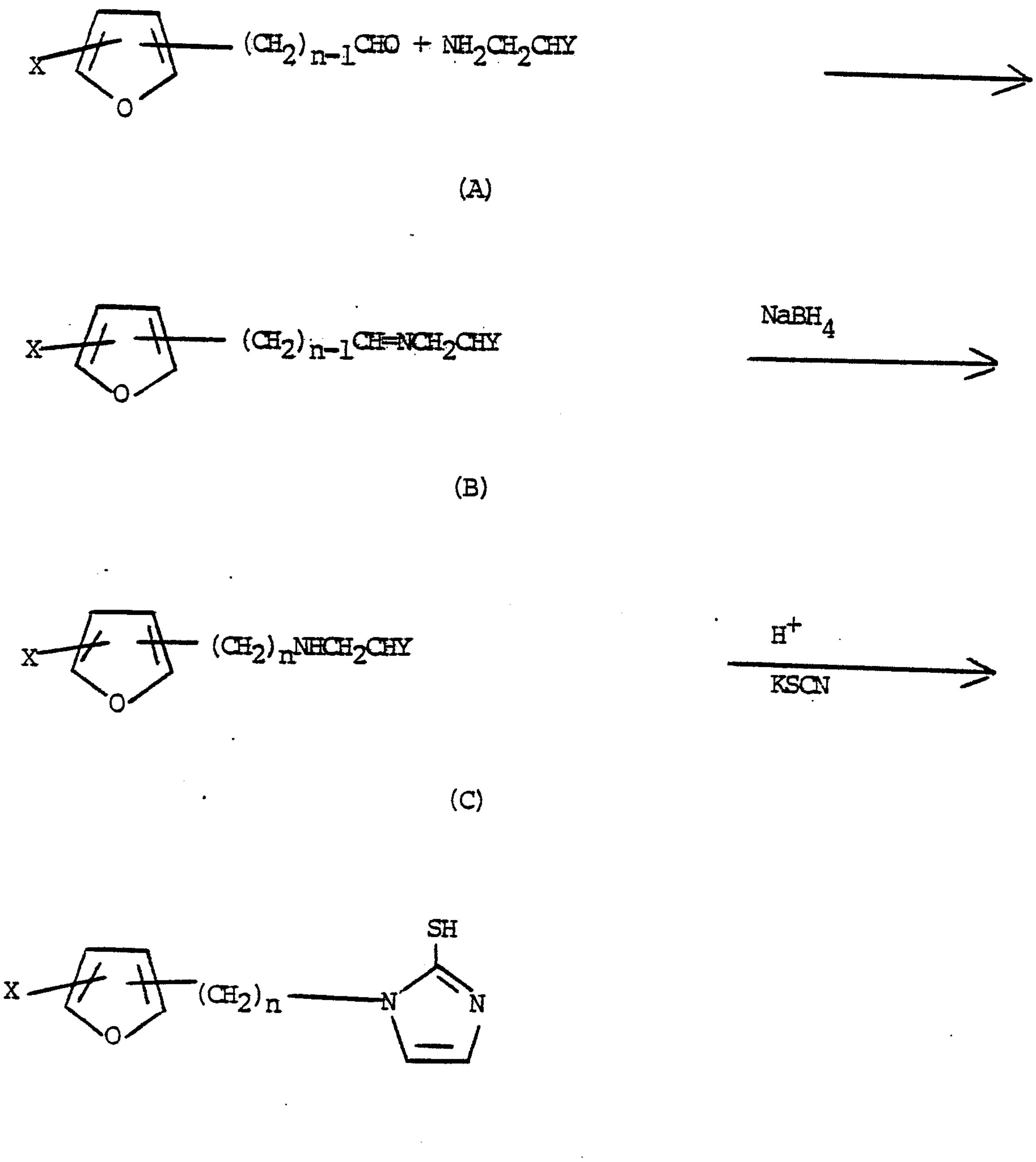


(A)

(B)

(C)

(I)

Scheme I illustrates reaction of furanylaldehyde and furanylalkylaldehydes (A) with an aminoacetaldehyde di($C_{1-4}$alkyl) acetal or ketal, preferably aminoacetaldehyde dimethyl acetal to form compounds of formula (B). This reaction is carried out by heating the reactants to 50 to 150°C, preferably 100°C, neat or in a suitable organic solvent such as diethyl ether, tetrahydrofuran, various lower alkyl alcohols, dimethylformamide, or ethyl acetate.

Formula (B) compounds then are reduced using a suitable reducing agent such as diborane, borane,

tetrahydrofuran, lithium aluminium hydride, hydrogen over platinum or palladium, or, preferably, sodium borohydride to prepare compounds of Formula (C).

Formula (I) compounds are prepared by reaction of formula (C) compounds with acidic thiocyanate, preferably potassium thiocyanate, in a suitable organic solvent such as listed above, preferably an aqueous $C_{1-4}$alkyl alcohol.

Compounds of the invention in which R is $C_{1-4}$alkyl are prepared by alkylating the corresponding Formula (I) compound where R is hydrogen with an alkyl halide, for example, methyl iodide in methanol, by known procedures. Other alkyl halides such as methyl bromide or methyl chloride, in appropriate solvents, can be substituted for methyl iodide. Further, the compounds in which R is an alkyl group other than methyl are prepared by substituting an alkyl halide such as butyl iodide, for the methyl halide to yield the desired substituted 1-(2-or 3-furanylalkyl)imidazole-2-alkylthiols of the invention.

In preparing the presently invented compounds of Formula I, novel intermediate compounds of the following formula were synthesized:

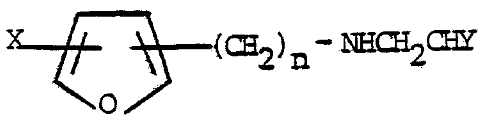

in which:

X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$, OR $CO_2C_aH_{2a+1}$ wherein a is 1-5, or any accessible combination thereof of up to 3 substituents; or

Y is $(OC_{1-4}alkyl)_2$ or $OC_{2-5}$alkylO; and

n is 1-5.

The pharmaceutically acceptable acid addition salts of compounds of the invention are formed with strong or moderately strong organic or inorganic acids by methods known in the art. For example, the base is reacted with an inorganic or organic acid in an aqueous miscible solvent such as ethanol with isolation of the salt by removing the solvent or in an aqueous immiscible solvent when the acid is soluble therein, such as ethyl ether or chloroform, with the desired salt separating directly or isolated by removing the solvent. Exemplary of the salts which are included in this invention are maleate, fumarate, lactate, oxalate, methanesulfonate, ethanesulfonate, benzenesulfonate, tartrate, citrate, hydrochloride, hydrobromide, sulfate, phosphate, quinate, and nitrate salts.

Because the Formula I compounds inhibit DBH activity, they have therapeutic value as diuretic, natriuretic, cardiotonic, antihypertensive, and vasodilator agents, as well as antiulcerogenic and anti-Parkinsonian agents. Listed in Table I are the compounds of the invention that were tested for in vitro DBH inhibition by a standard procedure for assaying conversion of tyramine to octopamine in the presence of DBH. J. J. Pisano, et al., Biochem. Biophys. Acta, 43, 566-568 (1960). Octopamine was assayed following sodium periodate oxidation to p-hydroxybenzaldehyde by measuring spectrophotometric absorbance at 330 nm. In Table I, inhibition is given in micromolar concentration of compound at which DBH activity was halved ($IC_{50}$). By this test, fusaric acid had an $IC_{50}$ of 0.8 micromolar.

Table I

| Compound | DBH $IC_{50}$ (μM) |
|---|---|
| 1-(2-furanylmethyl)imidazole-2-thiol | 56 |
| 1-(3-furanylmethyl)imidazole-2-thiol | 36 |

Further, spontaneously hypertensive rats were treated with a suspension or solution of 1-(2-furanylmethyl)imidazole-2-thiol at a dose of 50 mg/kg intraperitoneally, and mean arterial blood pressure was monitored for 260 minutes using indwelling cannulae in the tail arteries. When compared to vehicle-treated

controls, animals treated with the compound of the invention exhibited significant blood pressure reductions within approximately 30 minutes after treatment. Maximal blood pressure reduction was approximately 50 to 55 mm Hg.

The compounds of Formula I are incorporated into convenient pharmaceutical dosage forms such as capsules, tablets, or injectable preparations. Solid and liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glycerol monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical compositions are made following conventional techniques of a pharmaceutical chemist involving mixing, granulating and compressing, when necessary, for tablet forms, or mixing, filling, and dissolving the ingredients, as appropriate, to give the desired oral or parenteral products.

Doses of the present compounds of Formula I in a pharmaceutical dosage unit as described above will be an efficacious, nontoxic quantity selected from the range of 0.1-100 mg/kg of active compound, preferably 0.1-50 mg/kg. The selected dose is administered to a human patient in need of DBH inhibition from 1-6 times daily, orally, rectally, by injection, or continuously by infusion. Oral dosage units for human administration preferably contain from 1 to 500 mg of active compound. Parenteral administration, which generally requires lower dosages also can be used. Oral administration, at higher dosages, however, can be used when safe and convenient for the patient.

The method of this invention of inhibiting DBH activity in mammals, including humans, comprises administering internally to a subject in need of such inhibition an effective DBH inhibiting amount of a compound of Formula I.

The following examples illustrate preparation of Formula I compounds. The examples are not intended to limit the scope of the invention as defined hereinabove and as claimed below.

## EXAMPLE 1

1,(2-Furanylmethyl)imidazole-2-thiol

A mixture of aminoacetaldehyde dimethylacetal (5.45 ml, 0.05 mole) and furan-2-carboxaldehyde (4.6 g, 0.05 mole) was heated at 100°C for 2 hours and the reaction mixture was cooled in ice and diluted with ethyl alcohol (50 ml). Sodium borohydride (1 g) was added to the solution and the reaction mixture was stirred for 17 hours. The solvent was removed under vacuum, ethyl acetate (50 ml) was added, and the solution was washed with water and brine and dried over sodium sulfate. The mixture was filtered and the solvent was removed under vacuum. The resultant oil was diluted with ethyl alcohol (69 ml), potassium thiocyanate (5.59 g, 0.0575 mole) in water (115 ml) was added followed by concentrated hydrochloric acid (11.5 ml) and the solution was heated at reflux for 2.5 hours. The solvent was removed until an oil came out of solution at which point the reaction mixture was cooled in ice, diluted with water and the crude product was filtered. The product was first recrystallized from a mixture of ethyl acetate-hexane followed by ethyl acetate to give a solid melting at 109-110°C (1.28 g, 14%).

## EXAMPLE 2

1-(3-Furanylmethyl)imidazole-2-thiol

Following the above procedure, furan-3-carboxaldehyde gave 1-(3-furanylmethyl)imidazole-2-thiol as a solid melting at 115-116°C (1.35 g, 15%).

## EXAMPLE 3

1-(2-Furanylmethyl)imidazole-2-methylthiol

Reaction of 1-(2-furanylmethyl)imidazole-2-thiol, prepared as in Example 1, with methyl iodide in methanol by standard techniques yields 1-(2-furanylmethyl)imidazole-2-methylthiol.

EXAMPLE 4

1-(2-Furanylpropyl)imidazole-2-thiol

The process of Example 1 wherein furan-2-carboxaldehyde is replaced by 2-furanylpropanaldehyde yields 1-(2-furanylpropyl)imidazole-2-thiol.

EXAMPLE 5

1-(2-Furanylethyl)imidazole-2-thiol

The process of Example 1 wherein furan-2-carboxaldehyde is replaced by 2-furanylacetaldehyde yields 1-(2-furanylethyl)imidazole-2-thiol.

EXAMPLE 6

1-(3-Fluoro-2-furanylmethyl)imidazole-2-thiol

The process of Example 1 wherein furan-2-carboxaldehyde is replaced by 3-fluorofuran-2-carboxaldehyde yields 1-(3-fluoro-2-furanylmethyl)imidazole-2-thiol.

EXAMPLE 7

1-(4-Methyl-2-furanylmethyl)imidazole-2-thiol

The process of Example 1 wherein furan-2-carboxaldehyde is replaced by 4-methylfuran-2-carboxaldehyde yields 1-(4-methyl-2-furanylmethyl)imidazole-2-thiol.

EXAMPLE 8

1-(5-Nitro-2-furanylmethyl)imidazole-2-thiol

The process of Example 1 wherein furan-2-carboxaldehyde is replaced by 5-nitrofuran-2-carboxaldehyde yields 1-(5-nitro-2-furanylmethyl)imidazole-2-thiol.

EXAMPLE 9

1-(3-Ethoxy-2-furanylmethyl)imidazole-2-thiol

The process of Example 1 wherein furan-2-carboxaldehyde is replaced by 3-ethoxymethylfuran-2-carboxaldehyde yields 1-(3-ethoxy-2-furanylmethyl)imidazole-2-thiol.

EXAMPLE 10

1-(4-Acetoxy-2-furanylmethyl)imidazole-2-thiol

The process of Example 1 wherein furan-2-carboxaldehyde is replaced by 4-acetoxymethylfuran-2-carboxaldehyde yields 1-(4-acetoxy-2-furanylmethyl)imidazole-2-thiol.

EXAMPLE 11

1-(3,5-Dichloro-2-furanylmethyl)imidazole-2-thiol

The process of Example 1 wherein furan-2-carboxaldehyde is replaced by 3,5-dichlorofuran-2-carboxaldehyde yields 1-(3,5-dichloro-2-furanylmethyl)imidazole-2-thiol.

EXAMPLE 12

1-(3,5-Difluoro-4-methoxy-2-furanylmethyl)imidazole-2-thiol

The process of Example 1 wherein furan-2-carboxaldehyde is replaced by 3,5-difluoro-4-methoxy-furan-2-carboxaldehyde yields 1-(3,5-difluoro-4-methoxy-2-furanylmethyl)imidazole-2-thiol.

EXAMPLE 13

An oral dosage form for administering the presently invented compounds is produced by screening, mixing, and filling into hard gelatin capsules the ingredients in the proportions shown in Table III, below.

Table III

| Ingredients | Amounts |
|---|---|
| 1-(2-Furanylmethyl)imidazole-2-thiol | 50 mg |
| magnesium stearate | 5 mg |
| lactose | 75 mg |

EXAMPLE 14

The sucrose, calcium sulfate dihydrate, and Formula I compound shown in Table IV below, are mixed and granulated in the proportions shown with a 10% gelatin solution. The wet granules are screened, dried, mixed with the starch, talc and stearic acid, screened and compressed into a tablet.

## Table IV

| Ingredients | Amounts |
| --- | --- |
| 1-(3-Furanylmethyl)imidazole-2-thiol | 100 mg |
| calcium sulfate dihydrate | 150 mg |
| sucrose | 20 mg |
| starch | 10 mg |
| talc | 5 mg |
| stearic acid | 3 mg |

EXAMPLE 15

1-(3-Furanylmethyl)imidazole-2-thiol hydrochloride, 75 mg, is dispursed in 25 ml of normal saline to prepare an injectable preparation.

While the preferred embodiments of the invention are illustrated by the above, the invention is not limited to the precise instructions herein disclosed and that the right to all modifications coming within the scope of the following claims and all equivalents thereof is reserved.

## Claims

1. A compound of formula (I) :

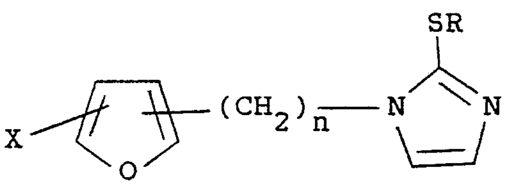

(I)

in which

X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$ or $CO_2C_aH_{2a+1}$ wherein a is 1-5, or any accessible combination thereof of up to 3 substituents;

R is H of $C_{1-4}$alkyl; and

n is 1-5; or

any pharmaceutically acceptable salt or hydrate thereof.

2. A compound of claim 1 wherein n is 1.

3. A compound of claim 2 wherein R is H.

4. A compound of claim 3 wherein $(CH_2)_n$ is at the 2 or 3 position of the furan ring.

5. A compound of claim 4 that is 1-(2-furanylmethyl)imidazole-2-thiol or 1-(3-furanylmethyl)imidazole-2-thiol.

6. A pharmaceutical composition comprising a pharmaceutical carrier and a compound of claim 1.

7. A pharmaceutical composition of claim 6 in a dosage unit form.

8. A pharmaceutical composition of claim 6 in which the compound of claim 1 is 1-(2-furanylmethyl)-imidazole-2-thiol or 1-(3-furanylmethyl)imidazole-2-thiol.

9. A compound as claimed in any one of claims 1 to 5 for use in therapy.

10. A compound as claimed in any one of claims 1 to 5 for use in inhibiting dopamine $\beta$-hydroxylase activity.

11. A compound as claimed in any one of claims 1 to 5 for use as an antihypertensive agent.

12. A process for the preparation of a compound of the formula (I) as defined in claim 1 which comprises cyclisation of a compound of the formula (II) :

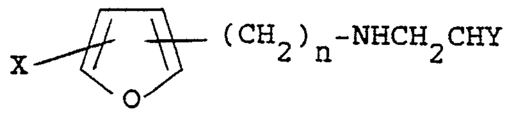

(II)

in which

X and n are as defined in claim 1; and

Y is $(OC_{1-4}alkyl)_2$ or $OC_{2-5}alkylO$;

by reaction with an acidic thiocyanate in the presence of an organic solvent and thereafter optionally, where R is H in the compound of formula (I) so formed alkylating to form a compound of the formula (I) in which R is $C_{1-4}$alkyl, and optionally forming any pharmaceutically acceptable salt or hydrate thereof.

13. A compound represented by the formula (II) :

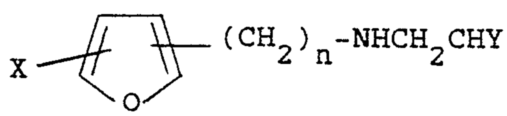

(II)

in which

X and n are as defined in claim 1; and

Y is $(OC_{1-4}alkyl)_2$ or $OC_{2-5}alkylO$.

Claims for the following contracting State: ES

1. A process for preparing a compound of the formula (I) :

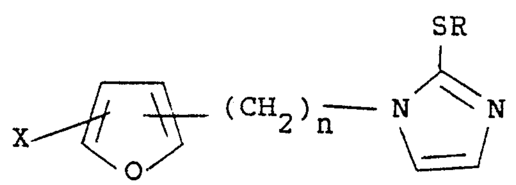

(I)

in which

X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$ or $CO_2C_aH_{2a+1}$ wherein a is 1-5, or any accessible combination thereof of up to 3 substituents;

R is H of $C_{1-4}$alkyl; and

n is 1-5; or

any pharmaceutically acceptable salt or hydrate thereof, which comprises cyclisation of a compound of the

formula (II) :

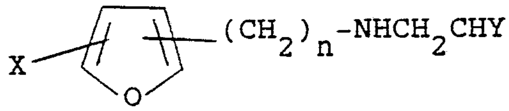

(II)

in which

X and n are as defined above; and

Y is $(OC_{1-4}alkyl)_2$ or $OC_{2-3}alkylO$ by reaction with an acidic thiocyanate in the presence of an organic solvent and thereafter optionally, where R is H in the compound of formula (I) so formed, alkylating to form a compound in which R is $C_{1-4}$alkyl and optionally forming any pharmaceutically acceptable salt or hydrate thereof.

2. The process of claim 1 in which the acidic thiocyanate is potassium thiocyanate.

3. The process of claim 1 or 2 in which the organic solvent is an aqueous $C_{1-4}$alkyl alcohol.

4. The process of claim 1 wherein in the compound of formula (I) n is 1.

5. The process of claim 4 wherein in the compound of formula (I) R is H.

6. The process of claim 5 wherein in the compound of formula (I) $(CH_2)_n$ is at the 2 or 3 position of the furan ring.

7. The process of claim 6 in which the compound of formula (I) is 1-(2-furanylmethyl)imidazole-2-thiol or 1-(3-furanylmethyl)imidazole-2-thiol.

8. A process for the preparation of a pharmaceutical composition which comprises bringing into association a pharmaceutical carrier and a compound of claim 1.

9. The process of claim 8 in which the pharmaceutical composition is in unit dosage form.

10. The process of claim 8 in which the compound of claim 1 is 1-(2-furanylmethyl)imidazole-2-thiol or 1-(3-furanylmethyl)imidazole-2-thiol.

11. A process for preparing a compound of the formula (II) :

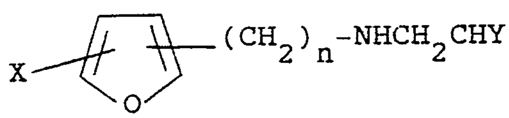

(II)

in which

X, n and Y are as defined in claim 1 which comprises reduction of a compound of formula (III) :

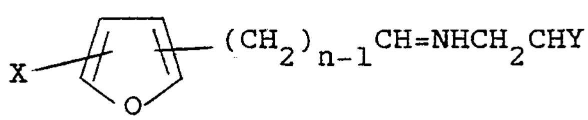

(III)

in which

X, n and Y are as defined in claim 1.

12. The process of claim 11 in which the reduction is carried out using sodium borohydride.

Claims for the following contracting State: GR

1. A process for preparing a compound of the formula (I) :

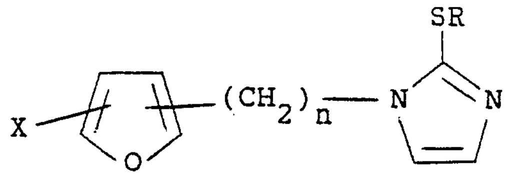

(I)

in which

X is H, F, Cl, Br, I, $C_{1-4}$alkyl, CN, $NO_2$, $SO_2NH_2$, COOH, CHO, $C_{1-4}$alkoxy, $CH_2OH$, $CF_3$, $SO_2CH_3$, $SO_2CF_3$ or $CO_2C_aH_{2a+1}$ wherein a is 1-5, or any accessible combination thereof of up to 3 substituents:

R is H or $C_{1-4}$alkyl; and

n is 1-5; or

any pharmaceutically acceptable salt or hydrate thereof, which comprises cyclisation of a compound of the formula (II) :

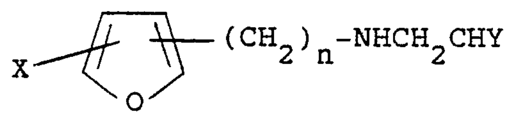

(II)

in which

X and n are as defined above; and

Y is $(OC_{1-4}alkyl)_2$ or $OC_{2-5}$alkylO by reaction with an acidic thiocyanate in the presence of an organic solvent and thereafter optionally, where R is H in the compound of formula (I) so formed, alkylating to form a compound in which R is $C_{1-4}$alkyl and optionally forming any pharmaceutically acceptable salt or hydrate thereof.

2. The process of claim 1 in which the acidic thiocyanate is potassium thiocyanate.

3. The process of claim 1 or 2 in which the organic solvent is an aqueous $C_{1-4}$alkyl alcohol.

4. The process of claim 1 wherein in the compound of formula (I) n is 1.

The process of claim 4 wherein in the compound of formula (I) R is H.

6. The process of claim 5 wherein in the compound of formula (I) $(CH_2)_n$ is at the 2 or 3 position of the furan ring.

7. The process of claim 6 in which the compound of formula (I) is 1-(2-furanylmethyl)imidazole-2-thiol or 1-(3-furanylmethyl)imidazole-2-thiol.

8. A process for the preparation of a pharmaceutical composition which comprises bringing into association a pharmaceutical carrier and a compound of claim 1.

9. The process of claim 8 in which the pharmaceutical composition is in unit dosage form.

10. The process of claim 8 in which the compound of claim 1 is 1-(2-furanylmethyl)imidazole-2-thiol or 1-(3-furanylmethyl)imidazole-2-thiol.

11. A process for preparing a compound of the formula (II) :

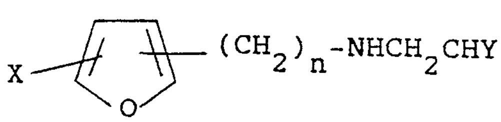

(II)

in which

X, n and Y are as defined in claim 1 which comprises reduction of a compound of formula (III) :

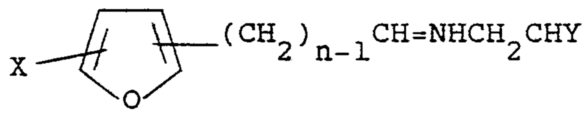

(III)

in which

X, n and Y are as defined in claim 1.

12. The process of claim 11 in which the reduction is carried out using sodium borohydride.

13. A compound of formula (II) :

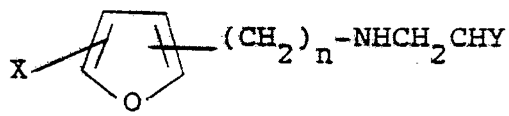

(II)

in which X, Y and and n are as defined in claim 1.